# EUROPEAN PATENT SPECIFICATION

(11) **EP 2 047 802 B1**
(45) Date of publication and mention of the grant of the patent: **03.10.2012**
(21) Application number: 08017677.9
(22) Date of filing: 08.10.2008
(51) Int. Cl.: A61B 8/00, G01S 15/89

(54) **Ultrasound diagnosis method and apparatus**
Verfahren und Vorrichtung zur Ultraschalldiagnose
Procédé et appareil de diagnostic ultrasonique

(30) Priority: 11.10.2007 JP 2007265622
(43) Date of publication of application: 15.04.2009
(73) Proprietor: FUJIFILM Corporation, Tokyo 106-8620 (JP)
(72) Inventor: Katsuyama, Kimito, Kanagawa (JP)
(74) Representative: Klunker . Schmitt-Nilson . Hirsch

(56) References cited:
- EP-A- 1 039 312
- JP-A- 2002 102 223
- JP-A- 2003 010 180
- ANDERSON M E ET AL: "The impact of sound speed errors on medical ultrasound imaging" JOURNAL OF THE ACOUSTICAL SOCIETY OF AMERICA, AIP / ACOUSTICAL SOCIETY OF AMERICA, MELVILLE, NY, US, vol. 107, no. 6, 1 June 2000 (2000-06-01), pages 3540-3548, XP012001761 ISSN: 0001-4966

## Description

### BACKGROUND OF THE INVENTION

### Field of the Invention

The present invention relates to an ultrasound diagnosis apparatus and method according to the preamble of claims 1 and 3, respectively, and particularly to an ultrasound diagnosis method and apparatus by which inter-frame, inter-portion similarity is determined in a stable, uniform manner.

### Description of the Related Art

Conventionally, ultrasound is used to acquire tomographic images of a subject for medical diagnosis. For example, when the motion of the heart or other organs is measured, "similarity" is used between frames to determine which portion in a frame corresponds to a certain portion in a previous frame and align these portions. As a conventional method for determining inter-frame, inter-portion similarity, a cross-correlation method, a normalized cross-correlation method, an SAD (source to axis distance) method, an SSD (source to surface distance) method, and other technologies are known. These technologies are used in a large number of fields, such as panoramic image synthesis, 3D image synthesis, motion/rotation correction, and speckle and tissue tracking.

For example, an apparatus according to the preamble of claim 1 is known in which one focuses on inter-frame change in texture to track probing positions without using a sensor (see Japanese Patent Application Laid-Open No. 2002-102223, for example). This technology is based on the fact that change in a speckle pattern that appears in ultrasound tomographic images with respect to shift in probing position is independent of the state of tissue being observed, and the change in a speckle pattern is used to identify the three-dimensional position of an ultrasound tomographic image.

That is, in this technology, a peak value of a correlation function for images before and after movement of an ultrasound probe is used to measure a movement vector in a tomographic image plane. A plurality of small areas are cut from one of the images before and after the movement. The peak position and the peak value of the correlation function for the areas and the other one of the images are used to measure the angle of rotation.

### SUMMARY OF THE INVENTION

The conventional technology described above that uses inter-frame similarity in texture can work in a more stable manner when the number of types of typically used texture is greater and work more accurately when the resolution of texture is higher. However, on the other hand, higher resolution of texture leads to greater inter-frame change, which disadvantageously reduces accuracy greatly.

Although the number of types of texture in a certain portion in an image can be increased by using a plurality of sets of RF data or amplitude images obtained by sending and receiving beams having different frequencies, extra transmission operations are disadvantageously necessary. Further, since a signal is divided, and the positional relationship between the probe and the reflector between transmission and reception operations is changed, the frame rate and resolution are lowered, and hence the accuracy is degraded.

The resolution of texture becomes lower when the discrepancy between the assumed sound speed and the actual sound speed during image generation is greater. Since the amount of discrepancy between the assumed sound speed and the actual sound speed varies from location to location, the resolution of texture or speckle varies from portion to portion, disadvantageously resulting in reduced accuracy in similarity and reduced uniformity, that is, accuracy varying from portion to portion.

Further, in a freehand-based 3D image reconstruction in the related art described above, a similarity value is used to estimate the distance over which the probe has moved. However, since the relationship between the similarity value and the distance over which the probe has moved varies with the resolution of speckle, accuracy in estimation disadvantageously decreases.

The present invention has been made in view of the above circumstances, and an object of the present invention is to provide an ultrasound diagnosis method and apparatus by which similarity can be determined in a stable, uniform, accurate manner without lowering the frame rate.

To achieve the above object, a first aspect of the present invention provides an ultrasound diagnosis apparatus including the features of claim 1.

Therefore, since a plurality of different speckle patterns based on assumed sound speeds are used, similarity can be determined in a stable manner. Further, since a variety of patterns from a low-resolution pattern to a high-resolution pattern are uniformly contained, similarity can be determined in a stable, accurate manner. Moreover, although an optimal sound speed (actual sound speed) is different from each portion, speckles with lower resolution to speckles with higher resolution are similarly contained, similarity can be uniformly determined in any portion.

The RF data or amplitude images are generated from received data produced by changing the assumed sound speed by multiple steps in a single transmission operation.

Therefore, since similarity is produced from received data obtained from the same transmission, the frame rate will not be lowered, and reduction in accuracy due to shift between transmission operations will not occur.

According to a preferred embodiment, in the ultrasound diagnosis apparatus the RF data or amplitude images are generated from data whose resolution in a direction in which the elements are arranged is higher than or equal to a distance between the elements.

Similarity can thus be accurately determined.

Similarly, to achieve the above object, a further aspect of the present invention provides an ultrasound diagnosis method including the steps defined by claim 3.

Therefore, since a plurality of different speckle patterns based on assumed sound speeds are used, similarity can be determined in a stable manner. Further, since a variety of patterns from a low-resolution pattern to a high-resolution pattern are uniformly contained, similarity can be determined in a stable, accurate manner. Moreover, although an optimal sound speed (actual sound speed) is different from each portion, speckles with lower resolution to speckles with higher resolution are similarly contained, similarity can be uniformly determined in any portion.

The RF data or amplitude images are generated from received data produced by changing the assumed sound speed by multiple steps in a single transmission operation.

Therefore, since similarity is produced from received data obtained from the same transmission, the frame rate will not be lowered, and reduction in accuracy due to shift between transmission operations will not occur.

According to a preferred aspect of the present invention, in the ultrasound diagnosis method, the RF data or amplitude images are generated from data whose resolution in a direction in which the elements are arranged is higher than or equal to a distance between the elements.

Similarity can thus be accurately determined.

As described above, according to the present invention, since a plurality of different speckle patterns based on assumed sound speeds are used, similarity can be determined in a stable manner. Further, since a variety of patterns from a low-resolution pattern to a high-resolution pattern are uniformly contained, similarity can be determined in a stable, accurate manner. Moreover, although an optimal sound speed (actual sound speed) is different from each portion, speckles with lower resolution to speckles with higher resolution are similarly contained, similarity can be uniformly determined in any portion.

### BRIEF DESCRIPTION OF THE DRAWINGS

Fig. 1 is a system configuration diagram showing a schematic configuration of an embodiment of an ultrasound diagnosis apparatus according to the present invention;
Fig. 2 is a flowchart showing the operation of an image generator;
Fig. 3 is a flowchart showing the flow of processes performed in a frame position calculator;
Fig. 4 is a flowchart showing the contents of processes performed in a display image generator;
Fig. 5 is a flowchart showing a first variation of processes performed in the frame position calculator; and
Fig. 6 is a flowchart showing a second variation of processes performed in the frame position calculator.

### DETAILED DESCRIPTION OF THE PREFERRED EMBODIMENTS

An ultrasound diagnosis method and apparatus according to the present invention will be described below in detail with reference to the accompanying drawings.

The present invention aims to use speckles in RF data or amplitude images generated at different assumed sound speeds to determine similarity in a stable, uniform, accurate manner without lowering the frame rate, and calculate frame positions.

With respect to the actual ultrasound speed (actual sound speed) transmitted toward a subject, ultrasound speeds set by changing an initial ultrasound speed stepwise by a predetermined amount multiple times are called set sound speeds or assumed sound speeds. The actual sound speed is also referred to as an optimal sound speed.

The present invention has been made in view of the following points: That is, (1) Since speckle patterns produced at different assumed sound speeds are different from one another, similarity is determined in a more stable manner by using the different assumed sound speeds. (2) Since speckle patterns produced at different assumed sound speeds are not only simply different from one another, but also uniformly contain a variety of speckle patterns from low-resolution speckle patterns to high-resolution speckle patterns, similarity is calculated in a more stable, accurate manner. (3) Although the discrepancy between an assumed sound speed and the optimal sound speed varies from location to location, using a plurality of assume sound speeds allows any portion to uniformly contain, in a similar manner to some extent, a variety of patterns from low-resolution patterns to high-resolution patterns. Similarity can therefore be calculated uniformly in any portion with greater accuracy. (4) Since similarity can be produced from received data obtained in the same single transmission, no extra transmission is necessary, and there is no shift between transmission operations.

A description will be made of freehand-based 3D image generation will be described below by way of example.

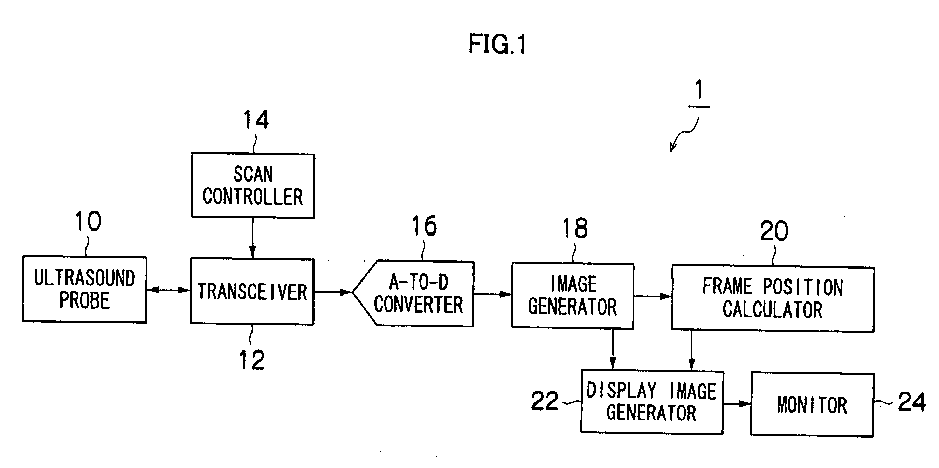

Fig. 1 is a system configuration diagram showing a schematic configuration of an embodiment of an ultrasound diagnosis apparatus according to the present invention.

As shown in Fig. 1, an ultrasound diagnosis apparatus 1 of the present embodiment uses ultrasound to capture and display ultrasound images of a site to be diagnosed in a subject. The ultrasound diagnosis apparatus 1 includes an ultrasound probe 10, a transceiver 12, a scan controller 14, an A-to-D converter 16, an image generator 18, a frame position calculator 20, a display image generator 22, and a monitor 24.

The ultrasound probe 10 transmits ultrasound toward a site to be diagnosed in the body of a subject and receives the ultrasound reflected off the body. The ultrasound probe 10 of the present embodiment includes a plurality of ultrasound transducers that form a one-dimensional ultrasound transducer array, and each of the ultrasound transducers is formed of an oscillator, for example, a PZT element or other piezoelectric elements with electrodes formed at both ends thereof. The electrodes are connected to the transceiver 12 via signal lines. When a voltage is applied to the electrodes, the oscillator produces ultrasound. The oscillator, when receiving reflected ultrasound, produces an electric signal and outputs it as a received signal.

The transceiver 12 sends an ultrasound transmission signal to the ultrasound probe 10 to cause the oscillators to produce ultrasound and transmit it based on a delay received from the scan controller 14. The elements in the ultrasound probe 10 receive reflected ultrasound and output the received signals, and the transceiver 12 then amplifies the received signals as they are (without performing reception focusing).

The A-to-D converter 16 receives the received ultrasound signals from the transceiver 12, AD-converts them, and sends the resultant signals to the image generator 18. In the image generator 18, the stored data received from the elements undergo reception focusing using delays based on variously set sound speeds (referred to as assumed sound speeds as compared to the actual sound speed transmitted to the subject, as described above), which will be described later in detail, and RF data based on the assumed sound speeds are produced.

The frame position calculator 20 uses a plurality of assumed sound speeds to calculate frame positions. The display image generator 22 uses the resultant images generated in the image generator 18 and the resultant frame positions calculated in the frame position calculator 20 to generate a 3D image to be displayed on the monitor 24.

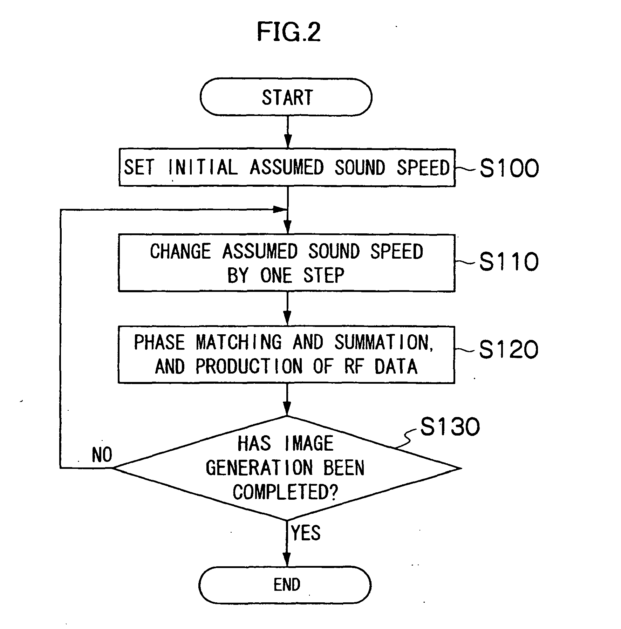

The operation of the image generator 18 in the configuration of the apparatus shown in Fig. 1 will be described with reference to the flowchart shown in Fig. 2.

The image generator 18 generates images from data obtained at variously changed assumed sound speeds.

First, in the step S 100 in Fig. 2, an initial assumed sound speed to be variously changed is set. The initial value is not limited to a specific one, but may be determined as appropriate, for example, 1400 [m/s].

Using the thus set initial value, the transceiver 12, which is under the control of the scan controller 14, transmits a signal to the ultrasound probe 10, which then acquires data based on the initial assumed sound speed and sends the data to the image generator 18.

In the step S 110, the assumed sound speed is changed by one step that corresponds to a predetermined amount, and the changed assumed sound speed is used to acquire ultrasound data. The predetermined amount of one step is not particularly limited to a specific value, but may be, for example, 20 [m/s], 10 [m/s], or 40 [m/s]. The assumed sound speed is then successively changed by the predetermined amount.

In the step S120, the resultant data based on the assumed sound speeds undergo phase matching and summation. RF (Radio Frequency) data are thus produced. The RF data contain amplitude information and phase information. The RF data is created by thus using images obtained at all the assumed sound speeds.

In the step S130, a judgment is made as to whether or not the image generation has been completed. When the image generation has not been completed, the control returns to the step S 110. In this case, the assumed sound speed is changed by one step, and the image generation continues. The image generation is judged to be completed when the above processes have been completed for all the assumed sound speeds. To this end, for example, the number of assumed sound speed changing steps to complete the image generation may be determined in advance, and a judgment is made as to whether the number is reached.

The operation of the frame position calculator 20 will be described.

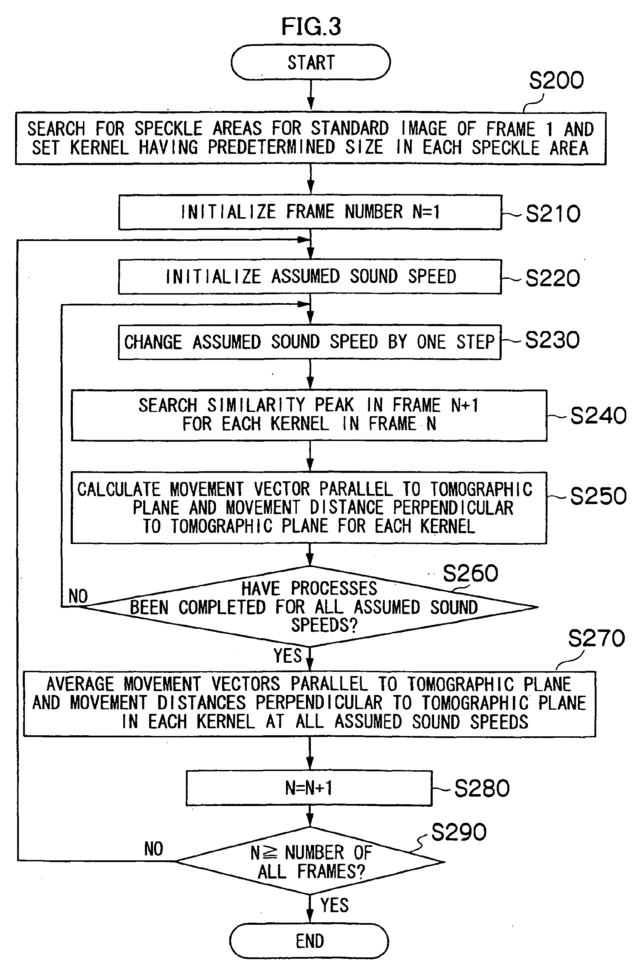

Fig. 3 is a flowchart showing the flow of processes performed in the frame position calculator 20.

First, in the step S200, speckle areas are searched for a standard image of the frame 1, and a kernel having a predetermined size is set in each of the detected speckle areas.

The standard image used to search for speckle areas is an image obtained at an ultrasound speed of 1540 [m/sec].

A method for judging whether or not searched speckle areas contain speckles is not limited to a specific one, but may be a known method for determining the degree of departure from the Rayleigh distribution. This method is based on the following fact: A speckle pattern that appears in an ultrasound image is a phenomenon in which when a large number of scatterers are distributed at a rate smaller than or equal to the resolution of ultrasound, a large number of processes of superposition of scatterers produce high-intensity and low-intensity portions in an ultrasound signal. When the scatterers are randomly distributed, the probability density distribution of amplitude values, which are the intensities of ultrasound signals reflected off the scatterers, follows the Rayleigh distribution expressed by P(x)=(x/s²)exp(-x²/2s²) (where s² represents dispersion and normalized as an average of zero). When a certain type of structures increases in tissue, however, the speckle pattern comes to reflect the structures and hence cannot be said to be random. As a result, the probability density function of brightness comes to depart from the Rayleigh distribution. Such a behavior is used to judge whether there are speckles.

Alternatively, a judgment may be made as to whether there are speckles by using a phase change characteristic in which when assumed sound speed is changed to produce RF data from received ultrasound images, the phase is random in the case of speckle irrespective of the assumed sound speeds.

Still alternatively, the user may specify speckle areas.

In the step S210, the frame number n is initialized to n=1. In the step S220, the assumed sound speed is initialized. As the initialized assumed sound speed, the data that has been obtained in the process performed in the image generator 18 may be used.

In the step S230, the assumed sound speed is changed by one step, and data at the resultant sound speed is acquired. To this end, again, the data that has been obtained in the process performed in the image generator 18 may be used.

In the step S240, a similarity peak is searched in the following frame n+1 for each kernel in the frame n, and a peak position and a peak value of the similarity peak are obtained.

A method for calculating similarity is not limited to a specific one. A cross-correlation method for calculating similarity ranging from 0 to 1 as an inter-frame cross-correlation coefficient between two sets of image data, an SAD method, or an SSD method can be used to calculate similarity. Data used to calculate similarity may be either amplitude images or RF data. The RF data used herein means data containing both amplitude information and phase information.

The similarity is a value indicating whether or not there is an unchanged portion between images. A high similarity indicates that the portion of interest in images does not change with time. That is, determining similarity shows which portion in a frame corresponds to a certain portion in the previous frame. Determining similarity thus allows the positions of the frames to be calculated.

In the step S250, for each kernel, the position of the similarity peak is used to determine a movement vector parallel to the tomographic plane (the amount of movement in the tomographic plane), and the value of the similarity peak is used to determine the movement distance perpendicular to the tomographic plane (the amount of movement perpendicular to the tomographic plane).

The relationship between the peak value and the amount of perpendicular movement is preferably measured (calculated) and tabulated in advance.

In the step S260, a judgment is made whether or not the above processes have been completed for all the assumed sound speeds. When the above processes have not been completed for all the assumed sound speeds, the control returns to the step S230, and the assumed sound speed is changed by one step to carry out the processes for the changed assumed sound speed.

When the above processes have been completed for all the assumed sound speeds, in the following step S270, the movement vectors parallel to the tomographic plane and the movement distances perpendicular to the tomographic plane in each kernel at all the assumed sound speeds are averaged.

Alternatively, the similarities in each kernel at all the assumed sound speeds may be averaged. That is, similarities may be averaged, and the averaged similarity and the similarity peak are used to determine the movement vector parallel to the tomographic plane and the movement distance perpendicular to the tomographic plane.

In the step S280, the frame number n is incremented by 1 to n+1.

In the step S290, a judgment is made whether or not the above processes have been completed for all the frames.

When the above processes have not been completed for all the frames, the control returns to the step S220, and the above processes are carried out for the following frame n+1. On the other hand, when the above processes have been completed for all the frames, the processes performed in the frame position calculator 20 are terminated.

The operation of the display image generator 22 will be described below.

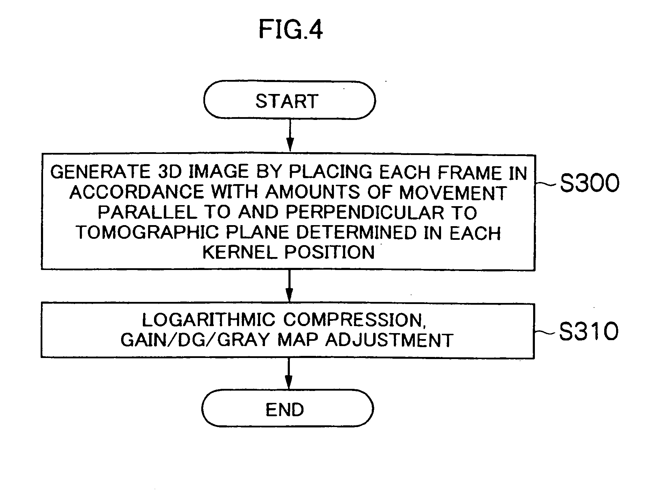

Fig. 4 is a flowchart showing the contents of processes performed in the display image generator 22.

First, in the step S300 in Fig. 4, a 3D image is generated by placing each frame in accordance with the amount of movement parallel to the tomographic plane and the amount of movement perpendicular to the tomographic plane determined in each kernel position in each frame.

In the step S310, typical logarithmic compression is performed on the generated 3D image, and gain/DR (dynamic range)/STC (sensitivity time control (depth weighting))/gray map adjustment and other operations are further performed.

The display image generated in the display image generator 22 is displayed on the monitor 24.

As described above, in the present embodiment, RF data or amplitude images generated at a plurality of different assumed sound speeds are used to determine similarity in each portion in each frame.

Since a plurality of different speckle patterns based on assumed sound speeds are used, similarity can be determined in a stable manner. In particular, since a variety of patterns from low-resolution patterns to high-resolution patterns are uniformly contained, similarity can be determined in a stable, accurate manner.

Further, although the optimal sound speed in a portion differs from those in other portions, a variety of patterns from low-resolution patterns to high-resolution patterns are similarly contained, similarity can be uniformly determined in any portion. Moreover, since similarity is produced from received data obtained in the same single transmission, the frame rate will not be lowered, and reduction in accuracy due to shift between transmission operations will not occur. Further, when the relationship between the similarity and the probe movement distance is used in freehand-based 3D image reconstruction, the relationship changes with the resolution of speckle. However, even in portions containing a variety of patterns from low-resolution patterns to high-resolution patterns, the relationship using these patterns is the same in any portion, whereby reduction in estimated accuracy due to different resolutions will not occur.

The processes performed in the frame position calculator 20 are not limited to those described above. Other exemplary processes performed in the frame position calculator 20 will be described below.

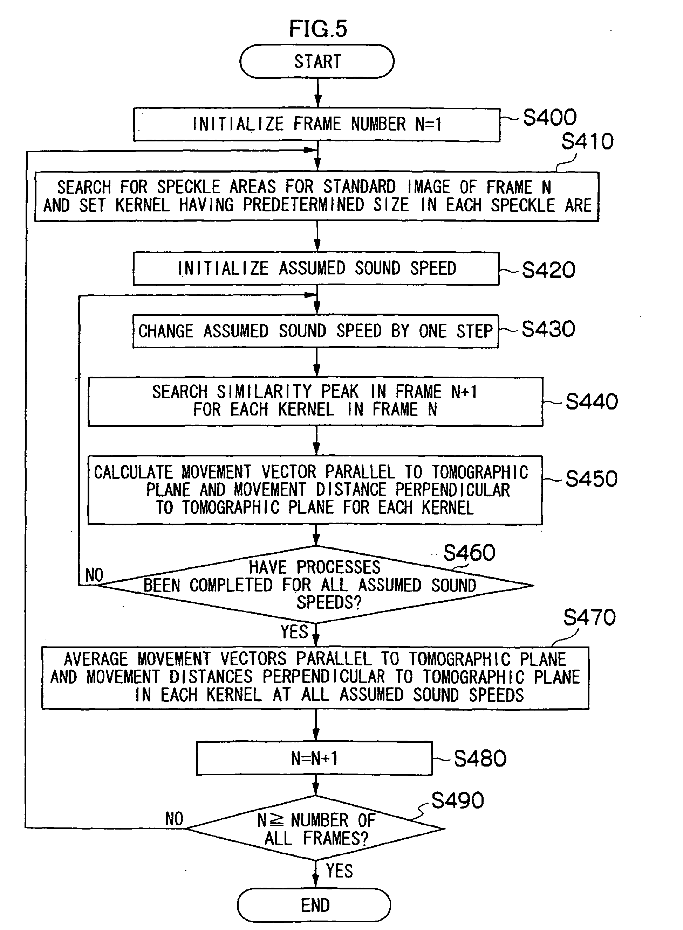

The flowchart in Fig. 5 shows a first variation of processes performed in the frame position calculator 20. In the processes shown in the flowchart in Fig. 3 described above, the speckle position is set in the first frame, but the speckle position is set in each frame in this example.

In the step S400 in Fig. 5, the frame number n is initialized to n=1.

In the step S410, speckle areas are searched for a standard image of the frame n, and a kernel having a predetermined size is set in each of the detected speckle areas. The standard image is an image obtained at an ultrasound speed of 1540 [m/sec].

In the step S420, the assumed sound speed is initialized, and in the step S430, the assumed sound speed is changed by one step.

In the step S440, a similarity peak is searched in the following frame n+1 for each kernel in the frame n.

In the step S450, for each kernel, a movement vector parallel to the tomographic plane and the movement distance perpendicular to the tomographic plane are calculated.

In the step S460, a judgment is made whether or not the above processes have been completed for all the assumed sound speeds. When the above processes have not been completed for all the assumed sound speeds, the control returns to the step S430, and the assumed sound speed is changed by one step to carry out the processes for the changed assumed sound speed.

When the above processes have been completed for all the assumed sound speeds, in the following step S470, the movement vectors parallel to the tomographic plane and the movement distances perpendicular to the tomographic plane in each kernel at all the assumed sound speeds are averaged.

In the following step S480, the frame number n is incremented by 1 to n+1, and in the step S490, a judgment is made whether or not the above processes have been completed for all the frames.

When the above processes have not been completed for all the frames, the control returns to the step S410, and the processes for the following frame n+1 are carried out. When the above processes have been completed for all the frames, the processes performed in the frame position calculator 20 are terminated.

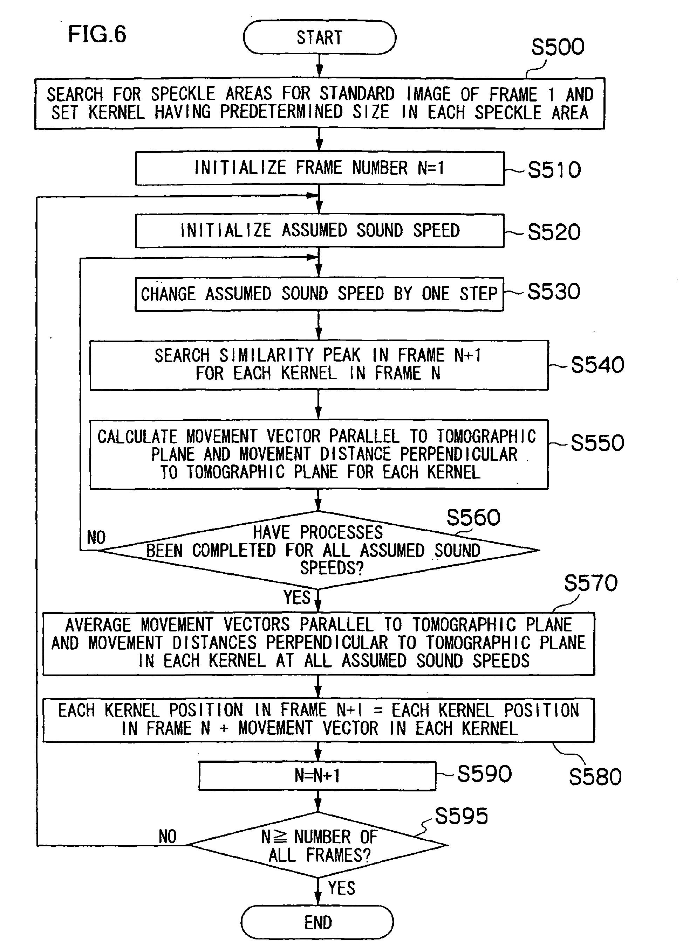

The flowchart in Fig. 6 shows a second variation of processes performed in the frame position calculator 20. In this example, the speckle initial position is set in the first frame, and the speckle position in each frame is set at a position to which the speckle position in the previous frame is moved in accordance with the movement vector.

First, in the step S500 in Fig. 6, speckle areas are searched for a standard image of the frame 1, and a kernel having a predetermined size is set in each of the detected speckle areas. The standard image is an image obtained at an ultrasound speed of 1540 [m/sec].

In the step S510, the frame number n is initialized to n=1. In the step S520, the assumed sound speed is initialized, and in the following step S530, the assumed sound speed is changed by one step.

In the step S540, a similarity peak is searched in the following frame n+1 for each kernel in the frame n.

In the step S550, for each kernel, a movement vector parallel to the tomographic plane and the movement distance perpendicular to the tomographic plane are calculated.

In the following step S560, a judgment is made whether or not the above processes have been completed for all the assumed sound speeds. When the above processes have not been completed for all the assumed sound speeds, the control returns to the step S530, and the assumed sound speed is changed by one step to carry out the processes for the changed assumed sound speed.

When the above processes have been completed for all the assumed sound speeds, the control proceeds to the following step S570, and the movement vectors parallel to the tomographic plane and the movement distances perpendicular to the tomographic plane in each kernel at all the assumed sound speeds are averaged.

In the step S580, the movement vector in each kernel is added to each kernel position in the frame n, and the result is set as each kernel position in the following frame n+1.

That is, (each kernel position in the following frame n+1)=(each kernel position in the frame n)+(the movement vector in each kernel).

In the following step S590, the frame number n is incremented by 1 to n+1, and in the following step S595, a judgment is made whether or not the above processes have been completed for all the frames. When the above processes have not been completed for all the frames, the control returns to the step S520, and the processes for the following frame n+1 are carried out. When the above processes have been completed for all the frames, the processes performed in the frame position calculator 20 are terminated.

As described above, the flow of processes performed in the frame position calculator 20 is not limited to a single flow, but several other flows are conceivable.

Recent software-based ultrasound apparatus and analog-based high-performance circuit configurations allow image generation at a variety of assumed sound speeds from a received signal obtained in the same single transmission operation. The apparatus in the present invention has a configuration necessary to obtain RF data or images at a variety of assumed sound speeds without shift between frames or not to lower the frame rate.

In a typical configuration of a conventional ultrasound apparatus, a display image is generated by forming transmission and reception beams at the sound ray positions spaced apart by the distance between elements, producing RF data or amplitude images, then interpolating them for sound rays between elements to produce amplitude data. In recent years, however, there is available a configuration in which transmission and reception beams are formed also for sound rays between elements and then RF data is produced.

In the apparatus configuration of the present embodiment, random change in speckle phase can be more accurately identified and similarity can be accurately calculated at a resolution in the scan direction higher than or equal to that obtained from the distance between elements, for example, by using high-resolution data in the scan direction so that data whose resolution in the scan direction is higher than or equal to that obtained from the distance between elements are used in the frame position calculator.

While in the above embodiment, the description has been made of a case where RF data obtained at one type of ultrasound transmission and reception frequency are used, the present invention encompasses a case where RF data obtained at a plurality of different frequencies of a fundamental wave and higher harmonic waves.

While the ultrasound diagnosis method and apparatus of the present invention have been described in detail, the present invention is not limited to the above example.

## Claims

1. An ultrasound diagnosis apparatus comprising:
an ultrasound probe (10) having a plurality of elements arranged therein, the ultrasound probe (10) transmitting ultrasound toward a subject, receiving an ultrasound signal reflected off the subject, and outputting a received signal; **characterized by**
a frame position calculation device (20) that determines similarity of each portion between *adjacent frames of* each consecutive frame in *respect to a plurality of* assumed sound speeds by using speckles in RF data or amplitude images based on two or more of the different assumed sound speeds out of the RF data or amplitude images produced *by changing the assumed sound speed by a predetermined value starting from a preset initial value with respect to a received signal obtained in a single transmission of the ultrasound,* and calculates frame positions by using *the similarity* in all assumed sound speeds.

2. The ultrasound diagnosis apparatus according to claim 1,
wherein the RF data or amplitude images are generated from data whose resolution in a direction in which the elements are arranged is higher than or equal to a distance between the elements.

3. An ultrasound diagnosis method comprising:
transmitting ultrasound form an ultrasound probe having a plurality of elements arranged therein toward a subject, receiving an ultrasound signal reflected off the subject and outputting a received signal **characterized by** the step of
determining similarity of each portion between *adjacent frames of* each *consecutive* frame in *respect to a plurality of* assumed sound speeds by using speckles in RF data or amplitude images based on two or more of the different assumed sound speeds out of the RF data or amplitude images produced *by changing the assumed sound speed by a predetermined value starting from a preset initial value with respect to a received signal obtained in a single transmission of the ultrasound,* and calculating frame positions by using *the similarity* in all assumed sound speeds.

4. The ultrasound diagnosis method according to claim 3,
wherein the RF data or amplitude images are generated from data whose resolution in a direction in which the elements are arranged is higher than or equal to a distance between the elements.

## Patentansprüche

1. Ultraschalldiagnosevorrichtung, umfassend:
eine Ultraschallsonde (10) mit einer Mehrzahl von darin angeordneten Elementen, wobei die Ultraschallsonde (10) Ultraschall in Richtung auf ein Objekt sendet, ein von dem Objekt reflektiertes Ultraschallsignal empfängt und ein empfangenes Signal ausgibt, **gekennzeichnet durch**
eine Einzelbildpositions-Berechnungseinrichtung (20), die eine Ähnlichkeit jedes Abschnitts zwischen benachbarten Einzelbildern von jedem aufeinanderfolgenden Einzelbild in Bezug auf eine Mehrzahl angenommener Schallgeschwindigkeiten unter Verwendung von Flecken von HF-Daten oder Amplitudenbildern basierend auf zwei oder mehr der verschiedenen angenommenen Schallgeschwindigkeiten derjenigen HF-Daten oder Amplitudenbilder bestimmt, die erzeugt werden **durch** Ändern der angenommenen Schallgeschwindigkeit um einen vorbestimmten Wert, beginnend bei einem voreingestellten Anfangswert, bezogen auf ein empfangenes Signal, welches bei einer einzelnen Sendung des Ultraschalls erhalten wird, und die Einzelbildpositionen berechnet unter Verwendung der Ähnlichkeit bei sämtlichen angenommenen Schallgeschwindigkeiten.

2. Ultraschalldiagnosevorrichtung nach Anspruch 1,
bei der die HF-Daten oder Amplitudenbilder erzeugt werden aus Daten, deren Auflösung in einer Richtung, in der die Elemente angeordnet sind, größer oder gleich ist einer Distanz zwischen den Elementen.

3. Ultraschalldiagnoseverfahren, umfassend:
Senden von Ultraschall aus einer Ultraschallsonde mit einer Mehrzahl von darin angeordneten Elementen in Richtung auf ein Objekt, Empfangen eines von dem Objekt reflektierten Ultraschallsignals und Ausgeben eines empfangenen Signals, **gekennzeichnet durch** den Schritt des Bestimmens einer Ähnlichkeit jedes Abschnitts zwischen benachbarten Einzelbildern von jedem aufeinanderfolgenden Einzelbild in Bezug auf eine Mehrzahl angenommener Schallgeschwindigkeiten unter Verwendung von Flecken von HF-Daten oder Amplitudenbildern, basierend auf zwei oder mehr der verschiedenen angenommenen Schallgeschwindigkeiten, von den HF-Daten oder Amplitudenbildern, die erzeugt werden **durch** Ändern der angenommen Schallgeschwindigkeit um einen vorbestimmten Wert, beginnend bei einem voreingestellten Anfangswert in Bezug auf ein empfangenes Signal, welches erhalten wird bei einem einzelnen Senden des Ultraschalls, und des Berechnens von Einzelbildpositionen unter Verwendung der Ähnlichkeit bei sämtlichen angenommenen Schallgeschwindigkeiten.

4. Ultraschalldiagnoseverfahren nach Anspruch 3,
bei dem die HF-Daten oder Amplitudenbilder erzeugt werden aus Daten, deren Auflösung in einer Richtung, in der die Elemente angeordnet sind, größer oder gleich ist einer Distanz zwischen den Elementen.

## Revendications

1. Appareil de diagnostic ultrasonique comportant :
une sonde ultrasonique (10) possédant une pluralité d'éléments disposés à l'intérieur, la sonde ultrasonique (10) transmettant des ultrasons vers un sujet, recevant un signal ultrasonique réfléchi par le sujet, et délivrant un signal reçu ; **caractérisé par**
un dispositif de calcul de position de trame (20) qui détermine une similarité de chaque partie entre des trames adjacentes de chaque trame consécutive par rapport à une pluralité de vitesses de son présumées en utilisant des bruits de speckle dans des données RF ou des images d'amplitude en fonction de deux vitesses de son présumées différentes ou plus parmi les données RF ou les images d'amplitude produites en changeant la vitesse de son présumée d'une valeur prédéterminée en commençant par une valeur initiale prédéterminée par rapport à un signal reçu obtenu lors d'une transmission unique d'ultrasons, et calcule les positions de trame en utilisant la similarité dans toutes les vitesses de son présumées.

2. Appareil de diagnostic ultrasonique selon la revendication 1,
dans lequel les données RF ou les images d'amplitude sont générées à partir de données dont la résolution dans une direction dans laquelle les éléments sont disposés est supérieure à ou égale à une distance entre les éléments.

3. Procédé de diagnostic ultrasonique comportait
la transmission d'ultrasons à partir d'une sonde ultrasonique possédant une pluralité d'éléments disposés à l'intérieur vers un sujet, la réception d'un signal ultrasonique réfléchi par le sujet et la délivrance d'un signal reçu, **caractérisée par** l'étape consistant à
déterminer une similarité de chaque partie entre des trames adjacentes de chaque trame consécutive par rapport à une pluralité de vitesses de son présumées en utilisant des bruits de speckle dans les données RF ou des images d'amplitude en fonction de deux vitesses de son présumées différentes ou plus parmi les données RF ou les images d'amplitude produites en changeant la vitesse de son présumée d'une valeur prédéterminée en commençant par une valeur initiale prédéterminée par rapport à un signal reçu obtenu lors d'une transmission unique d'ultrasons, et calculer des positions de trame en utilisant la similarité dans toutes les vitesses de son présumées.

4. Procédé de diagnostic ultrasonique selon la revendication 3,
dans lequel les données RF ou les images d'amplitude sont générées à partir de données dont la résolution dans une direction dans laquelle les éléments sont disposés est supérieure à ou égale à une distance entre les éléments.
